# EUROPEAN PATENT SPECIFICATION

(11) **EP 2 043 502 B1**
(45) Date of publication and mention of the grant of the patent: **12.03.2014**
(21) Application number: 06758065.4
(22) Date of filing: 13.07.2006
(51) Int. Cl.: G06F 19/00, A61N 1/372, A61N 1/362

(54) **INFORMATION MANAGEMENT IN DEVICES IMPLANTED IN A PATIENT**
INFORMATIONSMANAGEMENT BEI VORRICHTUNGEN, DIE IN EINEM PATIENTEN IMPLANTIERT WERDEN
GESTION D'INFORMATIONS RELATIVES À DES DISPOSITIFS IMPLANTÉS DANS UN PATIENT

(43) Date of publication of application: 08.04.2009
(73) Proprietor: St. Jude Medical AB, 175 84 Järfälla (SE)
(72) Inventor: HEDBERG, Sven-Erik, S-196 32 Kungsängen (SE); MALMBERG, Patrik, S-112 38 Stockholm (SE); LYCHOU, Leif, S-163 45 Spånga (SE); KERSTNA, Jürgen, S-165 60 Hässelby (SE); TJÄLLDIN, Ellen, S-112 25 Stockholm (SE)
(74) Representative: Sjölander, Henrik
(86) International application number: PCT/SE2006/000883
(87) International publication number: WO 2008/008008

(56) References cited:
- WO-A2-2006/035351
- WO-A2-2006/067217
- WO-A2-2006/109072
- DE-A1-102004 007 712
- US-A1- 2003 017 576
- US-A1- 2003 204 132
- US-A1- 2003 206 116
- US-A1- 2003 206 116
- US-A1- 2004 015 058
- US-A1- 2004 122 488
- US-A1- 2004 152 993
- US-A1- 2005 159 787
- US-A1- 2005 182 389
- US-A1- 2006 238 331
- US-B1- 6 289 238
- US-B1- 6 346 886

## Description

### TECHNICAL FIELD

The present invention generally relates to providing restricted and selective information management in devices adapted for physical connection to the animal or human body.

### BACKGROUND

Devices adapted for physical connection to a body of an animal or human user have become more and more usual during the last decades and find applications within different technological fields, in particular within the healthcare field. For example, different diagnostic and/or medical devices are designed to be connected to the human body and worn over a relatively long period of time for exerting their intended diagnostic and/or medical function. A typical example could be a glucose sensor that has been designed for being worn by a diabetic user and continuously and/or at selected time instances measures the blood glucose level of the user. Implantable medical devices (IMDs), such as pacemakers, implantable cardioverters or implantable defibrillators, are further examples of devices designed for physical connection to, in this case implantation into, the body of the user.

Several of these devices also include functionality for managing data associated with the user and/or the device. Thus, a diagnostic device typically includes a memory for storing the diagnostic data collected by the device during the time it is worn by and connected to the user. In this context, IMDs may be worth mentioning since these implantable devices typically have rather extensive memories for storing operational data of the device, diagnostic and medical data collected by the device and other data pertaining to the operation of the device and general condition of the patient having the IMD. This data forms part of the medical record of the patient and is therefore collected and used by the patient's physician in the patient surveillance. It has even been suggested in the art, see for example U.S. Patent No. 5,722,999, that the complete historical medical file of an IMD patient is stored in compressed form in the IMD.

Thus, there is a trend in the art to utilize devices worn by a user, in particular IMDs, that contain sensitive data of the patient and the device. This of course constitutes a security risk in terms of a malicious person might want to have access to this sensitive data of a given patient.

For example, the IMDs implanted in different patients are generally exchanged every few years. The removed IMD might then contain sensitive medical data of the patient's medical condition and/or health history, which should not fall in the hands of any person besides the patient and his/her physician(s). This in turn requires extensive administrative routines and security management when handling the explanted IMDs. Even if high administrative and security routines are present, it is almost impossible to guarantee that any data stored in the explanted IMD will not reach an unauthorized person.

The same problem exists for other devices which are physically connected to and worn by the user and in which sensitive data can be stored or entered.

A similar security problem exists in these devices if any person would be allowed to enter information into and/or update information stored in the devices. For example, an IMD typically has functionality for communicating with an external, non-implanted, device, e.g. programmer or physician workstation, by means of wireless communication. This is useful since the clinician may use the external device to program the operational parameters of the IMD, e.g. by modifying the pacing mode of the IMD after implantation.

However, unauthorized entering of sensitive data into the devices and or changing the data stored in the devices when connected to or implanted into the body of a user can cause major risks for the user in terms of addition of erroneous data in the medical patient history and incorrect settings of the operational device parameters. Similar problems exist for implantable memory chips and identification cards, the identification content of which should not be changeable after implantation.

Thus, there is a problem in the art in the management of sensitive data contained in devices connected to and worn by users.

US 2003/0206116 A1 discloses a medical monitoring system with a central station adapted to receive vital signs data concerning several patients. A patient monitor monitors a patient and collects vital signs data therefrom and communicates this data to the central station. The patient monitor also informs the central station of the identity of the particular patient to enable association of the vital signs data received from the patient monitor with the particular patient at the central station.

DE 10 2004 007 712 A1 discloses a sensor device that senses vital parameters from a human patient. The sensor device has a sensor element with a contact surface that is configured to be positioned on the skin of the human patient. The collected vital parameters are transmitted to a data collecting or processing device.

WO 2006/067217 A2 relates to a skin mountable medical device adapted to ensure that the device is properly in place with respect to the patient's body. Sensor means of the device is adapted to detect a property that can be indicative of a problematic condition relating to the interface of the device with the patient.

US 6,346,886 discloses an electronic identification apparatus having data storage memory on board a removable transceiver device having a processor and a transponder for receiving information pertaining to the person which it is attached and for storing the information in memory. If the transceiver has been removed from a base the processor performs a lockdown operation to prevent the stored data from being used in connection with another person.

### SUMMARY

The present invention overcomes these and other drawbacks of the prior art arrangements.

It is a general object of the present invention to provide management of information in devices adapted for physical connection to a body of a user.

It is another object of the invention to provide conditional information management in devices adapted for physical connection to a body of a user.

It is a particular object of an embodiment of the present invention to provide conditional external access to infonnation provided by an implanted device based on whether the device is implanted in the user body.

These and other objects are met by the invention as defined by the accompanying patent claims.

Briefly, the present invention involves a device adapted for physical connection to a body of a user. The device includes an information manager that is adapted for managing, such as providing or generating, retrieving from a memory or entering into a memory, information associated with the device or the user wearing the device. The management operation of this information manager is, according to the present invention, made dependent on whether the device is actually correctly physically connected to the user body. For this purpose the device comprises or has access to a sensor arrangement for sensing whether a physical device-body connection is present. In addition, the sensor arrangement generates a signal representative of the sensed physical-device connection (or lack thereof). The information manager is responsive to this generated signal and performs its information management function based on the signal.

In an embodiment the device is a medical device arranged on or in the user body for exerting a diagnostic or therapeutic function. In such a case, the sensor arrangement can be provided for sensing whether the device exerts its intended diagnostic and/or therapeutic function and generating the signal based on the sensing operation. This means that in this case the sensor arrangement investigates whether the device is functionally connected to the user body in such a way that it can perform the diagnostic/therapeutic function.

The device is an implantable device, such as an implantable medical device, which is designed for implantation into the body of a user. The sensor arrangement can then sense whether the device is correctly implanted in the user body or not and generating the signal based on this implantation sensing. This means that the data management is preferably only allowed for a fully functioning implantable device, e.g. retrieval of sensitive medical data or security attributed from a memory provided in the implantable device should not be possible if the device is explanted.

The sensor arrangement is preferably arranged for performing the decision and signal generation based on (physical) parameter data measured and collected from the user body. The sensor compares the measured data with a defined default value or default range that is representative of a correct physical connection. Thus, if the measured value corresponds to the default value or falls within the default range, the sensor arrangement considers the device to be physically connected to the user body. Examples of such parameter values include, temperature, lead impedance of a heart probe, intra cardiac signals, oxygen levels, respiratory signals, light measurement data, etc. It is anticipated that the sensor arrangement can measure and sense multiple different parameter values.

In an example not forming part of the invention, the sensor arrangement generates an access signal if it detects a physical connection to the user body. This access signal is forwarded to the information manager, which becomes activated. The manager can, for example, retrieve sensitive medical data from a device memory or generate security attributes based on the access signal. The provided information (medical data or security attributes) is typically transmitted from the device to an external unit, which requested the information. This embodiments therefore protects sensitive data and only distributes it to other units when the device is correctly worn by (such as implanted in) the user and prevents accessing the information once the device is removed from the user body.

Received information that is intended to be entered in the device, e.g. in a medical history memory, can also be conditional on whether the device is physically connected to the user or not. For example, certain data types, such as serial and model number of the device, should generally not be updatable once the device is arranged on the intended user. In such a case, the sensor arrangement can generate a restriction signal if it detects the presence of the device-body connection. The information manager is responsive to this restriction signal by preventing entry of the received information on the intended storage location.

The sensor arrangement can be configured for performing the sensing operation once the device receives a request for information or receives information to be entered in the device. Alternatively, or in addition, the sensor arrangement can be scheduled to perform the sensing operation at multiple different time instances. The sensor may have access to a sensing schedule, a clock circuit or some other equipment that triggers when a connection sensing, typically by measuring a body or device parameter value, should be performed. It the device includes diagnostic/therapy equipment which itself includes arrangement for measuring and monitoring different parameters, these parameter measurement results can be used by the sensor in the sensing operation.

If the sensor arrangement does not, based on the measured data, sense any physical connection between the device and the user body, the sensor preferably generates a lock signal. The information manager is then responsive to this lock signal by, at least temporarily, lock its information managing function. This means that the manager will not retrieve or generate any requested information once it has received a lock signal. Such a solution prevents the risk of someone stealing the device and connects it to his/her body for the purpose of maliciously get hold of the sensitive data in the device.

The invention can offer the following advantages:
- Reduces the risk of a malicious person getting hold of sensitive data from or entering erroneous data in a device adapted for physically connected to a user body;
- Enables a user to store and retrieve useful data in a medical/diagnostic device worn by or implanted into the user;
- Can be used as an important tool for facilitating integration of medical patient data in a distributed healthcare system by securely storing and securely accessing key information required in the data integration in implanted devices; and
- Can be used as an important tool for automation of secure access to sensitive data by securely storing and securely accessing key information required in the remote data access in implanted devices.

Other advantages offered by the present invention will be appreciated upon reading of the below description of the embodiments of the invention.

### SHORT DESCRIPTION OF THE DRAWINGS

The invention together with further objects and advantages thereof, may best be understood by making reference to the following description taken together with the accompanying drawings, in which:
Fig. 1 is a schematic overview of an embodiment of an information management system according to the present invention;
Fig. 2 is a schematic overview of an example of an information management system not forming part of the invention;
Fig. 3 is a flow diagram of an information management method not forming part of the invention;
Fig. 4 is a flow diagram of an information provision method not forming part of the invention;
Fig. 5 is a flow diagram of an additional step of the method of Fig. 4;
Fig. 6 is a flow diagram of an additional step of the method of Fig. 4;
Fig. 7 is a flow diagram illustrating an example of the providing step in Fig. 4 in more detail;
Fig. 8 is a flow diagram illustrating another example of the providing step in Fig. 4 in more detail;
Fig. 9 is a flow diagram illustrating an example of the sensing step in Fig. 4 in more detail;
Fig. 10 is a flow diagram illustrating an information management method according to an embodiment of the present invention;
Fig. 11 is a flow diagram illustrating an information management method not forming part of the invention;
Fig. 12 is a schematic block diagram of a device providing restricted and selective information management according to an embodiment of the present invention;
Fig. 13 is a schematic block diagram of a medical device providing restricted and selective information management according to an embodiment of the present invention;
Fig. 14 is a schematic block diagram of an implantable medical device providing restricted and selective information management according to an embodiment of the present invention;
Fig. 15 is a schematic block diagram of a device providing restricted and selective information management;
Fig. 16 is a schematic block diagram of an embodiment of an information manager according to the present invention;
Fig. 17 is a schematic block diagram of another example of an information manager not forming part of the invention; and
Fig. 18 is a schematic overview of transmission of information retrieved according to an embodiment of the present invention.

### DETAILED DESCRIPTION

Throughout the drawings, the same reference characters will be used for corresponding or similar elements.

The present invention relates to restricted and selective management of data stored in or to be stored in devices designed for physical connection to a body of an animal or human and designed for being worn by the animal or human for a period of time.

The present invention teaches a device adapted for physical connection to a body of a user and where the device is further adapted for containing and/or generating data and information associated with at least one of the user and the device. The information can have been entered in the device in connection with the manufacturing of the device, such as serial and model number, after manufacturing but before connection to the user body and/or after connection to the user. Alternatively, the information is generated by the device per se, for example, when requested by an external unit. The invention provides a solution for granting restricted access to the information in the device, restricted access to adding information to the device and/or restricted access to updating information stored in the device. This restricted access is realized by determining access only whether certain conditions are fulfilled, namely granting access depending on whether the device is actually connected to user body and worn by the user. Thus, the present invention senses and investigates whether the device is in correct operational environment, i.e. physically (mechanically) connected to the intended living user, before granting access to the data or information memory for the purpose of retrieving information therefrom, adding information thereto and/or updating information therein and/or generating and providing information.

Such a solution solves the prior art problems of unauthorized access to sensitive information and/or the security risks of maliciously adding erroneous information to the sensitive information when the device is operationally connected to the user body. For example, access to sensitive information stored in the device will only be granted if the device is physically connected to the body of the user. Thus, once the device is removed from the user body, the sensitive information stored therein is no longer accessible and retrievable from the device. Correspondingly, addition of new information to the device could be granted only if the device is connected to the user body or denied if the device is in physical connection to the body, depending on the type of information to be entered. For example, information and data that typically should be static for a given user and device, such as user identifier and device identifier (serial and model numbers), could be added only if the device is not connected to the user body.

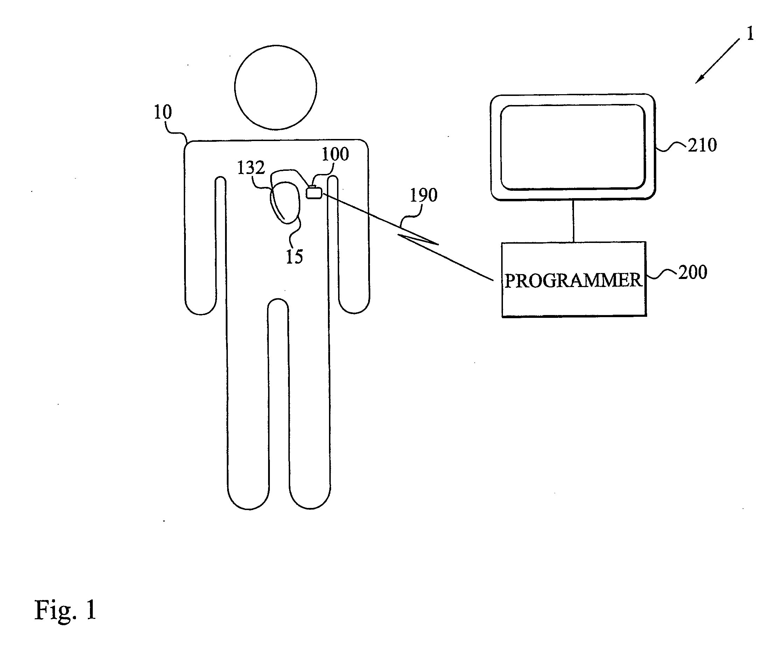

Fig. 1 is an overview of an information management system 1 according to the present invention. The system 1 includes at least one device 100 designed for physical connection to the body 10 of a user. In this embodiment, the device is in the form of an implantable medical device, IMD, 100 implanted in a patient or subject body 10 in need thereof. In Fig. 1, the IMD 100 is illustrated as a device that monitors and/or provides therapy to the heart 15 of the patient 10, such as a pacemaker, defibrillator or cardioverter. However, the present invention is not limited to cardiac-associated IMDs but may also be practiced with other implantable medical devices, such as drug pumps, neurological stimulators, physical signal recorders, oxygen sensors, or the like.

The IMD 100 preferably includes functionality for collecting and sampling physiological and diagnostic data, such as intracardic electrogram (IEGM) and/or event marker data in the case of a cardiac-associated IMD, which is schematically illustrated by the IMD lead or probe 132. This physiological data and also data representing operational parameters and settings of the IMD 100 might be useful for a clinician and is therefore preferably stored in the IMD 100. This stored patient and/or device data can then be communicated to an external device 200. As a consequence, the IMD 100 typically includes a communications module to communicate with the external device 200 via a wireless link 190. This wireless transmission could generally be realized using any known non-invasive wireless technique usable in medical applications. A preferred example is radio frequency (RF) telemetry, in which radio packets carrying data are transmitted over the wireless link 190 between the IMD 100 and the external device 200. Other examples of useful wireless transmission techniques are inductive data transmission.

The external device 200 of the system 1 is represented by a communicating data processing device in Fig. 1. This data processing device 200 could generally include functionality for monitoring the operation of the IMD 100 based on operational parameters and routine status reports received from the IMD 100. The processing device 200 can also include programmer functionality allowing downloading or programming of the operational parameters of the IMD 100, e.g. by modifying the pacing mode of the IMD 100 after implantation. As was noted in the foregoing, the IMD 100 might also upload physiological data, to the data processing device 200 over the communications link 190. The processing device 200 then processes the data e.g. in order to find any deviation from the normal patient state. Alternatively, or in addition, the physiological data received from the IMD 100 can be processed by the processing device 200 for presentation on an associated display screen 210.

The retrieval and transmission of the medical data collected by the IMD 100 is made, in this embodiment of the present invention, dependent on whether the IMD 100 is implanted into the patient body 10 or not. Thus, the IMD 100 preferably comprises or is at least connectable to a sensor for sensing whether the IMD 100 is actually implanted in the patient body 10. In this context, different physical parameters can be measured and monitored in order to determine whether the IMD 100 is implanted or not, which is discussed in more detail herein. The sensor is typically connected to the lead 132 or the lead 132 constitutes a part of the sensor. The sensor then utilizes measurement results collected by the lead 132 for asserting that the device 100 is actually implanted.

If the sensor determines, based on the measurement result(s), that the IMD 100 is actually implanted it generates an access signal. A memory manager in the IMD 100 that manages the retrieval and entering of data and information in the IMD memory is responsive to this access signal and retrieves, in this embodiment, data from the memory based on the access signal.

Thus, in a typical implementation, the patient 10 visits a physician that wants to collect the medical data generated by the IMD 100 since a last visiting. The physician then uses his/her workstation 200 for wirelessly transmitting an information request to the IMD 100. Alternatively, the IMD 100 can be configured for automatically transmitting information, if correctly implanted, without reception of any external request. In either case, the request is typically forwarded to the memory manager that retrieves the requested information from the memory if and only if a correct access signal has been generated by the sensor, which corresponds to that the IMD 100 is implantable in the patient body 10.

The sensor can perform the investigation of whether the IMD 100 is implanted in the body 10 upon reception of the information request from the physician workstation 200. Alternatively, the sensor performs the investigation at different time instances independent on whether an information request has bee received or not.

The memory manager retrieves the information if it has received an access signal and the retrieved information is forwarded to the requesting workstation 200 and can be used by the physician, e.g. displayed on the screen 210.

This means that if the IMD 100 is subsequently explanted and a malicious person would try to extract the information stored in the IMD 100 using a programmer, workstation or any other external unit 200 that can communicate and exchange data with the IMD 100, the sensor in the IMD 100 will not generate any access signal. This in turn means that the memory manager, upon receiving the information request, will not retrieve the requested information from the memory since the IMD 100 is no longer implanted, as determined based on no correct access signal.

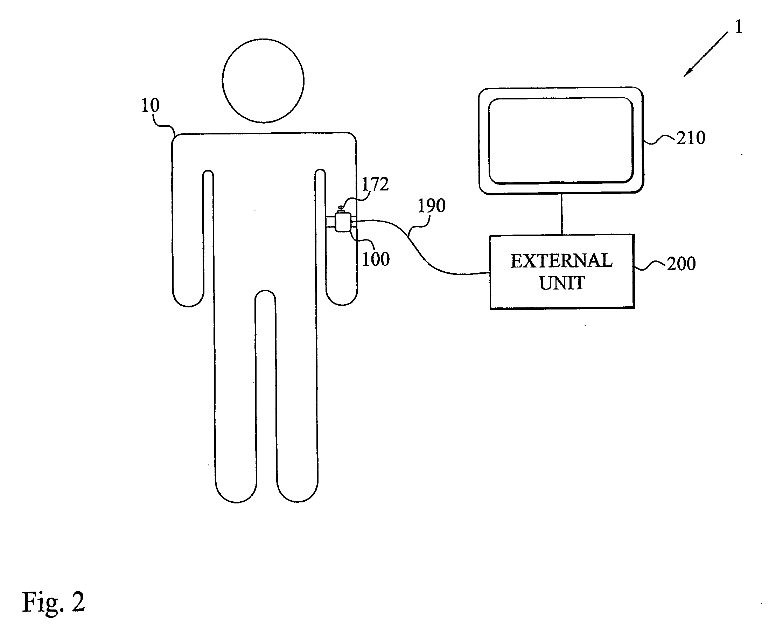

Fig. 2 is a corresponding overview of another information management system 1. In this case, the device 100 is not an IMD but is instead physically and mechanically connected to the user body 10. The device 100 is typically connected to and worn by the user for exerting some diagnostic and/or medical function to the patient body 10. The device 100 comprises or at least is connectable to a probe/sensor head 172 for sensing whether the device 100 is actually physically connected to the body 10.

Similar to Fig. 1, external access to information stored in the device 100 or generated by the device 100 will only be granted if the device 100 is physically connected to the user body 10 and the sensor 172 generates the access signal. If physically connected, the data can be wirelessly or wiredly transmitted 190 to an external requesting unit 200 in correspondence with Fig. 1.

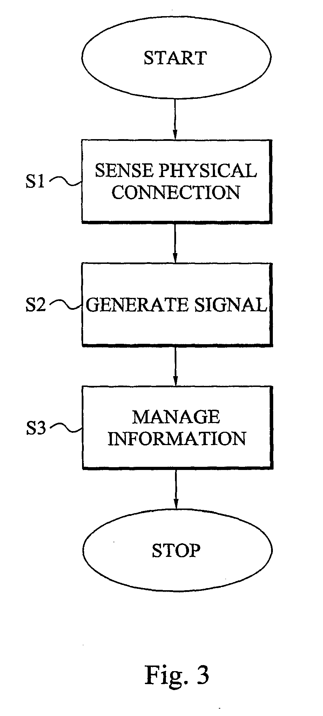

In Fig. 3, an information management method is provided in a device physically connectable to a body of a living user. This method starts in step S1, which involves sensing whether the device is physically connected to the body. This sensing step S1 can be performed according to different embodiments. Firstly, the sensing can be once, e.g. in connection with reception a request for provided information or reception of new information to be added to and stored in the device. Alternatively, the sensing is performed at multiple different time instances, e.g. periodically at selected time instance, such as every X₁ ms, every X₂ s, every X₃ minute, every X₄ hour, etc., where X₁, X₂, X₃, X₄ are positive numbers. The sensor in the device then preferably comprises or has access to a clock unit or some other equipment for determining when to perform a sensing operation. In certain embodiments, the sensor can be arranged for more or less continuously sense whether the device is physically connected to the user body. However, in real life using digital circuitry, a continuous sensing is typically performed by sensing at multiple discrete sampling or time instances.

Thus, the sensing step S1 can be performed once, but is preferable performed at multiple time instance, such as performed periodically, intermittently or continuously.

The sensing of step S1 determines whether the device is physically or mechanically connected to the device. In most applications, the device has at least one defined function to perform on the user body, such as a diagnostic function or a medical function. In such a case, the sensing step S1 preferably involves sensing whether the device is physically and functionally connected to the user body. Functionally connected should be interpreted as physically connected to (on the outside of or implanted into) the user body in such a way that the device can correctly perform its intended (diagnostic/medical) function. For example, a diagnostic glucose sensor can be arranged onto the patient body and include a probe inserted into a blood vessel of the patient for measuring the glucose level in the blood. This glucose sensor is then functionally connected if arranged on the patient body and the probe is in contact with the circulating blood. Another example is a pacemaker having both diagnostic and therapeutic functionality. The pacemaker typically includes at least one lead inserted into the patient's heart and used for monitoring the heart activity and function of the patient and for applying hear pacing (therapy) if needed. A functional connection can then be present if the lead is correctly implanted in the patient's heart.

In a typical embodiment of the present invention this sensing step comprises measuring or at least estimating, based on measurements performed in or on the user body, a parameter value in the body. This parameter value is then compared with a pre-defined default value or a default value range representative of correct physical connection of the device to the user body. For example, body temperature of a human is generally around 37 °C. Thus, the device can include or be connected to a thermometer for temperature measurements. If the measurement results indicate a temperature of around 37 °C, such as within the interval 32 - 42 °C, preferably 34 - 40 °C, and more preferably 35 - 39 °C, a physical connection between the device and the human body is assumed. Thus, the sensing of the present invention can be based on events generated by the body in/on which the device is arranged, such as heart beat or temperature changes.

In this context, multiple parameters can be measured and used in the sensing step S1. For example, temperature measurements can be complemented with, or replaced by, a more (cardiac) functional parameter, such as measured blood glucose level and normal cardiac activity. Thus, when discussing sensing a physical connection between device and body this includes performing the sensing based on measurements of one or more physical parameters in the user body.

Examples of suitable physical parameters that can be used in the sensing step S 1 include, but are not limited to, measured body temperature, lead impedance of an electrode system, typically for IMDs and pacemakers, different activity signals, such as pulmonary/respiratory activity, cardiac activity, etc. Generally any parameter that can be measurable in the body and that can be representative of a physical implementation or connection of a device in or to the user body can be used according to the present invention. In the present invention, the device is an IMD and the measured parameter is selected from at least one of: IMD temperature (should generally equal to body temperature), lead impedance of the electrode system (should generally be equal to normal impedance value), different intracardiac and implantation signals.

In a next step S2, a (access/restriction/lock) signal is generated. This signal is representative of whether a physical connection between the device and the user body was sensed in step S1. In a first example, the signal is generated if a physical connection between the device and body was sensed. In a first embodiment, the signal is generated if no physical connection between the device and the body was sensed. In a second embodiment, a first signal type is generated if a physical device-body connection exists and a second signal type is generated if no such connection is present.

A next step S3 manages information associated with at least one of the user and the device in the device based on or in response to the generated signal. In this context managing information includes providing information in/from the device, such as retrieving information and data from a memory or data storage, generating information in the device; adding information to the device, such as entering information in a memory or data storage of the device; updating information in the device, such as updating operational parameters and settings of the device. This information management is performed based on the generated access/restriction signal and will therefore be performed dependent on whether the device is correctly physically connected to the body or not.

The signal is a restriction signal, thus, denying access to provided information, denying access to addition or updating of information in the device if and only if a physical connection between the device and body has been sensed.

The restriction signal was generated if the device is physically connected to the user body. Alternatively, the signal is an access signal which grants access to provided information, grants access to addition or updating of information if the device is not physically connected to the user body.

The information management in step S3 is preferably performed based on a most previously generated access/restriction signal. This means that, if at a previous sensing occasion a physical connection was sensed, but no physical connection was sensed or detected at the very last sensing instance, the generated signal should preferably reflect this latest condition (no sensed connection). In this context, a maximum time period can be used for reflecting how up-to-date the generated signal is. If more than the maximum time period has elapsed since the last connection sensing, the information management can be put on hold until a more recent sensing has been performed. In such an embodiment, each generated signal is preferably associated with or assigned a time-to-live value or time stamp that is representative of the time instance of when the sensing operation was performed. If the time-to-live value of the newest generated signal has expired at the management occasion, a new sensing should be performed. This can be realized by actively requesting the sensor to perform the sensing or awaiting a next scheduled sensing.

The method then ends.

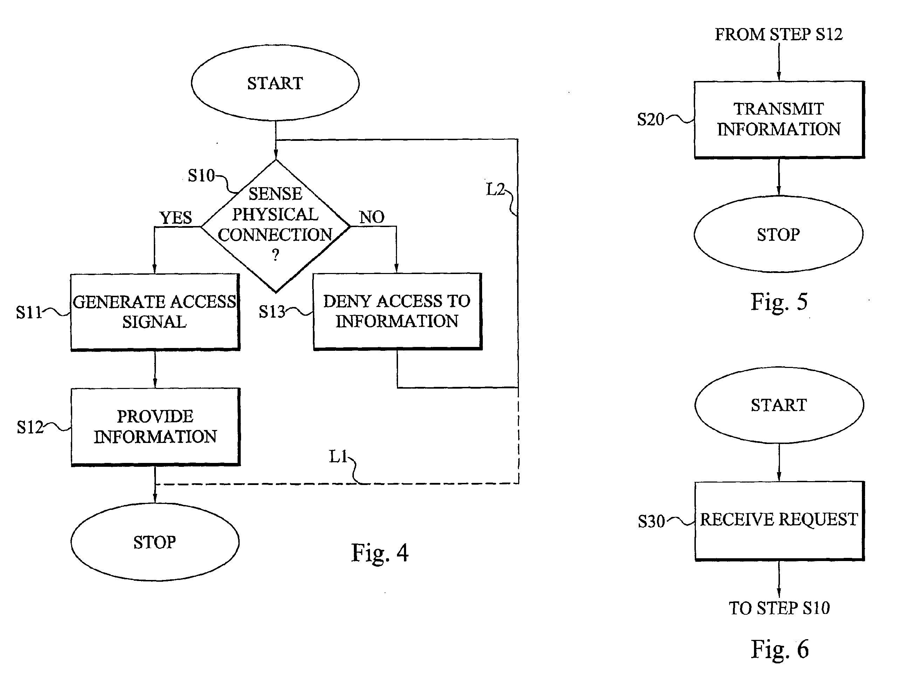

Fig. 4 is a flow diagram of an embodiment of the information management method of Fig. 3 and which illustrates an information provision method not forming part of the present invention.

The method starts in step S10, which involves sensing whether a physical connection is present between the device and the user body. This step basically corresponds to step S1 of Fig. 1 and is not further discussed. If a physical connection is sensed in step S10, the method continues to step S 11, where an access signal is generated. This access signal, thus, reflects the existence of a physical device-body connection. In a next step S12, the device provides information based on the access signal. This provided information is typically information requested from an external unit and will be sent thereto by the device.

If, however, no physical connection was sensed in step S10, the method continues to step S 13. Since no physical device-body connection is present access to the requested information is denied and no information is provided by the device. This can be realized by generating a restriction signal based on the detected lack of connection. The information provider implemented in the device is then responsive to the restriction signal and will not provide any information based on the restriction signal. If the information was requested by an external unit, an error message can be returned informing the external unit that access to the requested information was denied.

The sensing of step S10 is preferably performed at multiple time instances, as was discussed in the foregoing and which is schematically reflected by the lines L1 and L2.

Fig. 5 is a flow diagram of an additional step of the information providing method of Fig. 4. The method continues from step S12 of Fig. 4. In a next step S20, the provided information is transmitted to a requesting external unit. With reference to Figs. 1 and 2, the device 100 wirelessly (Fig. 1) or wiredly (Fig. 2) transmits 190 the provided information to the external unit 200, where the information can be stored, processed and/or displayed on a display screen 210. The method then ends.

Fig. 6 is a flow diagram of an additional step of the information providing method of Fig. 4. The method starts in step S30, where the device receives a request for information from an external requesting unit. This request can be sent wirelessly, by RF or inductive transmission, or wiredly. The request preferably comprises an identifier of the request information. Alternatively, the device is programmed to know what type information to send or simply transmits all information provided since a previous request instance.

The method then continues to step S 10 in Fig. 4. In this embodiment, the sensing operation in step S10 can be performed in response to reception of the information request. In such a case, the device performs a sensing operation when it receives a request. Alternatively, the sensing is made independent on the request reception and is instead performed at given scheduled time instances or continuously.

It is anticipated that in the communication of requests and information illustrated in Figs. 5 and 6, authentication and encryption/decryption procedures may be used. For example, the requesting external unit may have to authenticate itself in connection with requesting information from the device so that the device knows that the requesting unit is authorized to receive provided information. Correspondingly, information protection can be used using symmetric or private/public keys, especially when the provided information is transmitted wirelessly to the requesting unit. These procedures are well-known to the person skilled in the art and are not described further herein.

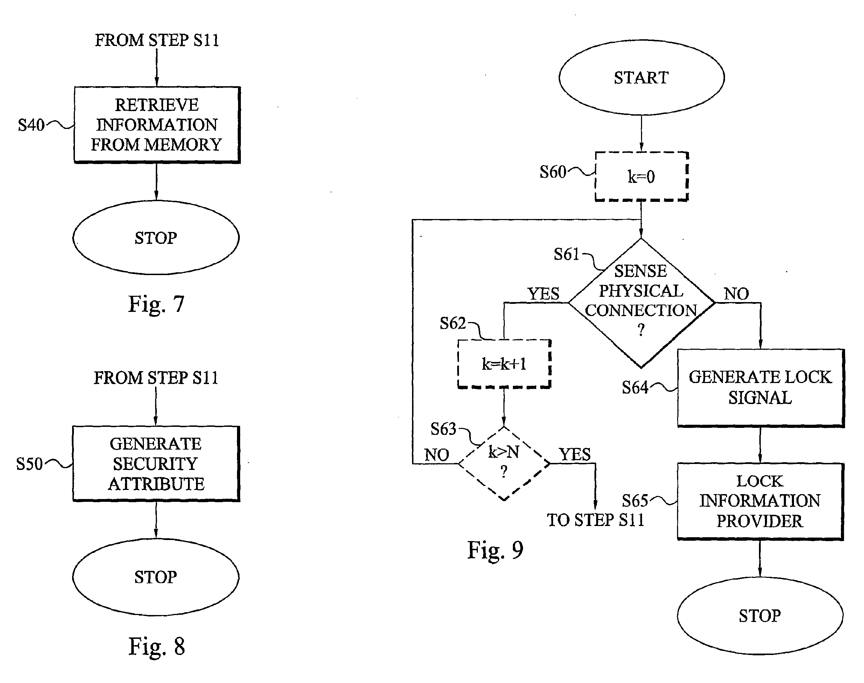

Fig. 7 is a flow diagram illustrating an example of the information providing step of Fig. 4 in more detail according to an embodiment. In this embodiment, the device comprises a data memory adapted for storing information/data associated with the device and/or the user to which the device is connected. This stored data can have been entered in the memory at the time of device manufacturing (typically serial and model number), after manufacturing but before connection to the user body (typically user identifier and original device settings) and/or after connection to the user (typically diagnostic or medical data collected by the device during operation).

The information stored in the memory can, thus, include medical data of the user generated by the device itself. This data includes, for example, medical results of different physical parameters the device is to monitor. Medical data of the patient but generated by other sources can previously have be sent to the device for storage therein. In this context it is actually possible to store the complete medical history of the patient in the device, or at least a portion thereof, see U.S. Patent No. 5,722,999.

The device can also include different security attributes useful when accessing and distributing medical data of the patient, to which the device is connected. For example, patient having an IMD or other medical device physically connected to his/her body is a user of a remote care service provide by a healthcare enterprise. The patient can posses a home monitoring unit used for interrogating the IMD and for requesting information therefrom. The home monitoring unit is furthermore able to connect to commodity network devices via standard ports like USB (Universal Serial Bus) or wireless. The patient wants to access the Web application provided by the enterprise from the commodity workstation and the application requires the patient to login, in order to identify the patient. This is important since the content pushed from the remote server to the patient's Web browser is patient specific. It is unreliable to expect the patient to be able to remember and type in his/her security credentials (login name and password). This problem can be solved by storing the user credentials in the IMD and preferably automatically uploading them therefrom during the login process. This eliminates the problems of patient's forgetting and/or mistyping the credentials.

In such a case, the home monitoring unit would interrogate the security credentials from the IMD and programmatically submit them to the system of the service provider, which in turn identifies the patient and allows the patient to access the Web application and his/her own private data. The credentials can also be used to identify the patient using the home monitoring unit to send medical data collected by the IMD to external servers for storage.

The access to these user credentials is preferably only allowed for a fully functioning IMD, i.e. it should not be possible to retrieve the credentials from an explanted device. Therefore, such security credentials is preferably stored or generated in an IMD and retrievable therefrom only if the IMD is implanted in (physically and functionally connected to) the patient.

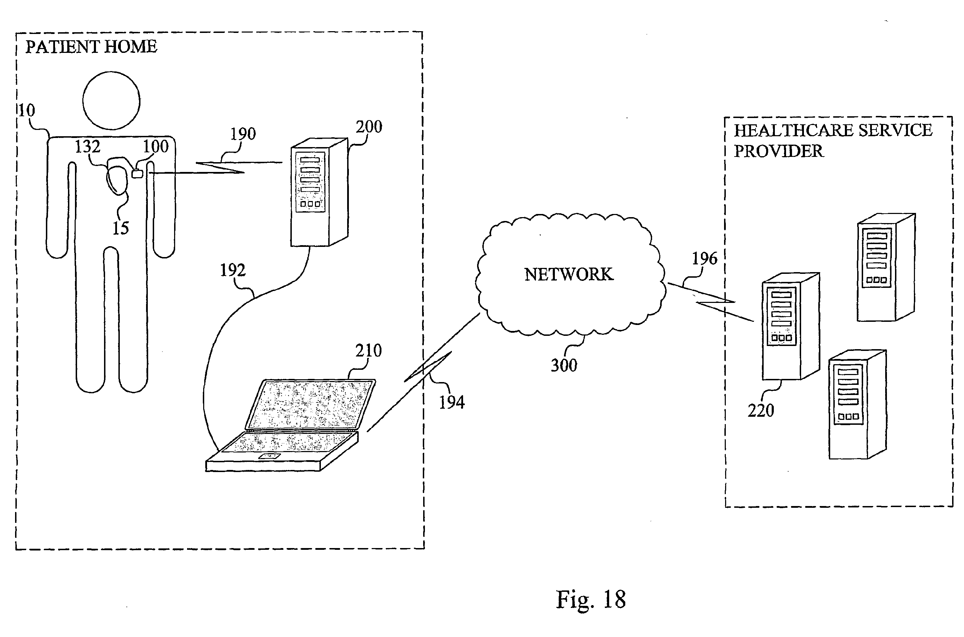

Fig. 18 schematically illustrates how retrieval of security credentials can be used. The patient 10 has an implanted medical device 100, illustrated as a pacemaker in the figure. This IMD 100 includes a memory for storing security credentials (login name and password) needed for accessing a Web application managed by a healthcare service provider. The patient 10 accesses the patient Web application using a home computer 210 that communicates with a server 220 of the service provider over a network 300, such as Internet. Instead of requiring manual supply of the user name and password, the Web application access a home monitoring unit 200 connected 192 (with wire 192 in the figure but could be wireless) to the computer 210, which the patient 10 used when accessing the server 220 from. The home monitoring unit 200 is instructed by the application to get the credentials from the IMD 100. The unit 200 transmits a credential request 190 to the IMD 100, which provides the credentials from a memory if an access signal has been generated, i.e. if and only if the IMD 100 is correctly implanted in the patient body 10 (as possible determined from measurements performed by the lead 132 inserted in the heart 15). The credentials are sent 190 to the home monitoring unit 200, which forwards 192 them to the computer 210. The computer then sends 194, 196 the credentials over the network 300 to the remote server 220. This allows the patient to access his/her own patient record stored in the server 220.

In a different scenario, the home monitoring unit 200 starts a routine follow-up procedure to generate medical or diagnostic data of the patient, possibly by means of the IMD 100. The generated data from the routine follow-up can then be entered into the patient record stored in the remote server 220. In such a case, security credentials stored in the IMD 100 are used, as described above, for accessing the server 220.

In a similar situation, a Certificate Authority issues certificates and keys for mutual authentication of connecting nodes. The healthcare systems of today become increasingly more mobile and networked. This means that a patient will be connected to healthcare provider systems in various different ways, which configuration is not known upfront and is in fact subject to frequent changes. In such a mobile environment, security becomes a risk and extensive authentication and data encryption is most often required. However, logistic of certificates and private keys is one of the biggest problems for reliable authentication. In addition, such a networked system must also deal with the Protected Health Information (PHI). This PHI includes any data in exchange records that would allow identification of the patient, or any other sensitive patient information which access is regulated by law or policies. Since such sensitive data is exchanged between networked systems, the PHI is preferably encoded with secret key.

Unauthorized access to these certificates and keys can be prevented if they are stored in a device, preferably an IMD, according to the present invention instead of in a programmer or some other external communications unit. Upon interrogation they can be temporarily copied to key store and trust store of the programmer/communications unit and used during the session. When the session is completed the certificates, keys and other Certificate Issued tokens are removed from the communications unit.

The access of the sensitive security attributes is only granted if the device (IMD) is physically and preferably functionally connected to (implanted in) the living user body. Thus, attributes should not be retrievable from an explanted IMD. By storing the CA tokens and secret keys in the device, a lot of administration can be avoided in maintaining and updating keys and the security attributes. In addition, the patient can use any computer or networking communications unit, having the capability of directly or indirectly communicating with the device, to access his patient records and still know that no one else can.

Returning to Fig. 18, the patient 10 would like to access the patient Web application on the remote server 220 using his/her home computer 210. CA issued tokens stored in the IMD 100 can then be fetched, if the IMD 100 is correctly implanted in the patient 10, by the computer 210 using the home monitoring unit 200. The patient's computer 210 is then authenticated by transmitting the CA tokens to the server 220. Since the tokens are stored in the IMD 100 and only accessible when the IMD 100 is actually implanted in the patient 10, only the patient 10 can access the server 220.

If the IMD 100 or home monitoring unit 200 has generated any patient data (PHI), a secret key stored in the IMD 100 and transiently retrievable therefrom only when the IMD 100 is implanted can be temporarily used by the home monitoring unit 200 for encrypting the sensitive data before it is transmitted by the computer 210 over the network to the healthcare service provider.

In another issue, one of the big problems today that hinder medical information to be shared among caregivers is the difficulty to associate medical records made by one healthcare provider with medical records made by another. There are physical barriers, but even when IT related issues are solved, different healthcare enterprises use different systems to identify the patients. These patient identifiers can vary between countries, regions, hospitals and even different departments of a given healthcare enterprise. Hospitals often have their own patient administration procedures and patient identifiers. When medical documents are received from another healthcare provider, the association to the same patient in the destination's system might be complicated. In worst case, a document is assigned to wrong patient, which may lead to wrong diagnosis, treatment, etc. Anyhow, the patient mapping from source to destination system is often a manual operation, thereby time consuming and error prone.

The present examples can be used for solving these problems by letting a patient carry information about his/her patient identifiers used in different systems that keep his/her medical records. Thus, the device physically connected to the patient, preferably implanted into the patient, contains a list, record or database of these different patient identifiers. In a preferred implementation, the list comprises, for each patient identifier entry, at least two components: i) the identifier of the person/patient and ii) an identifier of the healthcare enterprise/system who issued the identifier of the person.

Each time the patient visits a physician that wants to retrieve a medical document of the patient, recorded by and stored in another healthcare enterprise that uses another patient identifier, the physician can send, using his/her workstation, programmer or some other unit that can communicate with the device (see Figs. 1 and 2), a request for the identifier list. The device retrieves the list from an internal memory if the device is physically connected to the patient body and forwards it to the physician's workstation.

Correspondingly, once the patient visits a new healthcare enterprise that uses a hitherto non-employed identifier of the patient, a list update message comprising the newly assigned patient identifier and an identifier of the healthcare enterprise is transmitted to the device. This updated information could be conditionally entered in the list memory if and only if the device is actually physically connected to the relevant patient.

As was briefly noted in the foregoing, it is not uncommon for the complete medical patient history of a patient to be distributed among and stored in different healthcare enterprises and records. This can be a problem since when a patient comes to visit, the clinician often needs to lookup information from previous treatments, which may have taken place in different times at different healthcare enterprises. In most cases, the clinician has no overview of what has happened to the patient, other than what the patient is able to tell. This lack of information compromises the quality of care. In addition, identification and retrieval of medical documents from different healthcare enterprises takes a lot of time and effort from not only the current clinician but also from other healthcare providers who possess the required information, which adds cost to the healthcare service.

IHE (Integrating the Healthcare Enterprise) is an organization that makes vendors and clinicians to collaborate to define how medical data communication standards should be effectively used for solving data integration problems. Different integration profiles primarily address the need of patient data sharing cross different clinical users.

The medical/diagnostic device worn by the patient can constitute a part of the IHE system by storing a listing or containing a database of where different recorded medical documents of the patient are found. Thus, such a list includes information of the medical documents generated for the patient and location information, identifying which healthcare enterprise that stores the data or some other information that can be used for identifying from where the document can be fetched.

This document/location list is then preferably only retrievable and sent to a requesting unit if the device is correctly physically and preferably functionally connected to the intended patient body. Thus, when the patient visits a clinician that would like to obtain a document recorded at another healthcare enterprise, the clinician's workstation requests the device to provide the document list. A memory manager of the device retrieves the list in response to an access signal (which is representative of a correct physical device-body connection) from the connection sensor and the list is forwarded to the workstation. The clinician then uses the list for identifying the correct remote document location and can access, preferably electronically, the document therefrom. In this document retrieval, security attributes (credentials, CA tokens, etc.) described above can be fetched from the device and used by the clinician workstation.

It is also anticipated that the sensitive data stored in or provided from the device can include information of the device. For example, parameter and operational settings of a programmable device could be useful for a physician or device technician but constitutes valuable and sensitive data if ended up at a malicious non-authorized person. Therefore, such parameter settings are preferably only retrievable from the device when the device is physically connected to the intended user. Correspondingly, serial and/or model number of the device is preferably only retrievable therefrom when physically connected to the user body.

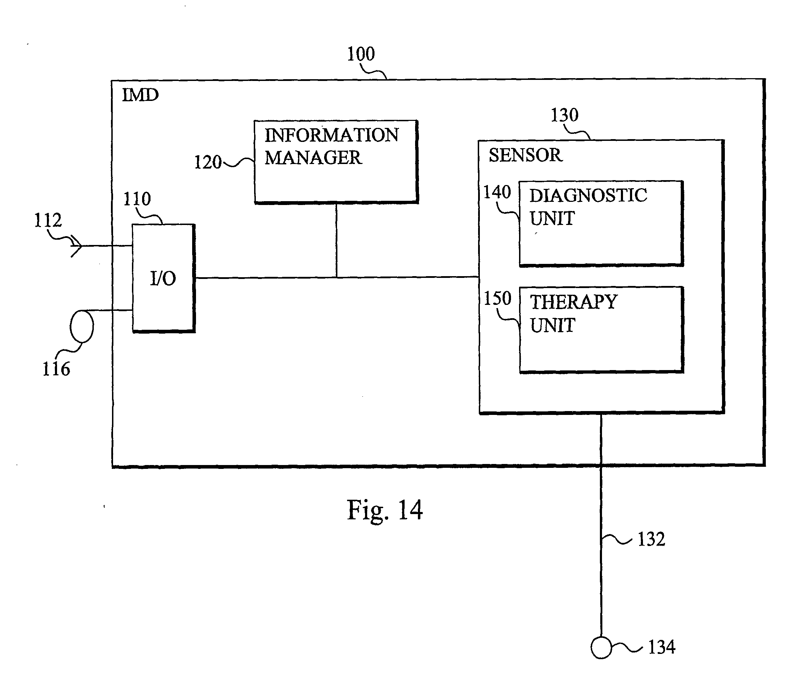

Fig. 8 is a flow diagram illustrating another embodiment of the information providing step of Fig. 14. The method continues from step S11 of Fig. 4. In a next step S50, the device generates security attributes based on the access signal. These generated security attributes can be used in the node-node authentication, session encryption/decryption, login procedures, etc. described in the foregoing.

Thus, security attributes are generated by the device when needed, similar to attribute generators used by the clients of Internet banks. The attributes are generated based on an access signal, i.e. only generated if the device is physically connected to the user body. Alternatively, the security attributes could be generated without any access signal input but will not be distributed from the device unless an access signal has been correctly provided.

In an extension of this concept, the key to unlock the security attribute generation can also be stored in the device and only be retrievable from the key storage if the device is physically connected to the user body (as determined based on the presence of an access signal).

In a preferred implementation, the body-device connection sensing is preferably performed more than once, such as at defined multiple, i.e. at least two, time instances, intermittently, periodically. In such a case, the access signal will be generated if the device is physically connected to the body at each of the multiple time instances. In addition, a lock signal is preferably generated if the device, during the sensing activity, is not physically connected to the body at one of the sensing occasions. The sensor of the device could then be responsive to this lock signal and is prevented, based on the lock signal, from generating any access signal even if the device subsequently becomes physically connected to a user body. Alternatively, the information provider in the device is responsive to the lock signal will not, once a lock signal has been generated, provide any requested information. This is in particular useful for devices that are not implantable into the body of the user. Measurement at multiple different time instances and possibly locking the device minimizes the risk of removing the device from the correct user body and installing it onto a non-intended user.

Fig. 9 is a flow diagram illustrating these aspects not forming part of the invention. The method starts in the optional step S60, where a sensing counter k is set to zero. This sensing counter k is used for counting the number of times a sensing operation has been performed by the device. The counter k is useful in those applications where an access signal is only generable if at least a minimum number N of sensing operations have been performed. The method continues to step S61, where the sensor senses whether a physical device-body connection is present. This step S61 basically corresponds to step S1 of Fig. 3 and S10 of Fig. 4 and is not further described. If a physical connection is sensed, the method continues to the optional step S62, where the sensing counter k is increased by one to reflect that a sensing operation has been performed and that a correct physical body-device connection has been sensed. The method continues with the optional step S63, where the sensing counter k is compared with the minimum number N. If the sensing counter is larger than the minimum number, the method continues to step S11 of Fig. 4, where the access signal is generated. If, however, the counter does not exceed the minimum number too few sensing operations have been performed for generating an access signal and the method continues to step S61, where a new sensing process can be started directly or awaiting a scheduled next sensing instance.

In another implementation, the optional steps S60, S62 and S63 are omitted so that an access signal can be generated even after a single sensing instance. However, the method preferably continues, after generation of access signal and provision of information (if required), back to step S61. This means that device is arranged for conducting multiple scheduled sensing operations throughout its operation and implementation to the user body.

If, however, at one of the performed sensing occasions, no physical connection between the device and user body is sensed, the sensor preferably generates a lock signal in step S64. This lock signal prevents the information provider, in step S65, from providing any information from the device and to an external requesting unit. In a first implementation, the sensor is responsive to the lock signal by being prevented from generating any access signals once a lock signal has been generated. In a second implementation, the information provider is responsive to the lock signal by being prevented from providing any information even if a subsequent access signal is generated.

This example, thus, minimizes the risk of removing the device from a correct user body and connecting the device to a non-intended body for the purpose of extracting sensitive information from the device. Thus, if, during such a fraudulence procedure, the device performs a connection sensing once the device is moved from the correct user to the malicious user, a lock signal will be generated and will prevent any provision of sensitive information from the device.

The method then ends.

In a possible implementation, the locked device could be reset by the device manufacturer, patient physician or some other trusted person. In such a case, the lock imposed by the lock signal is reversed and the device can be operated as usual again by extracting information therefrom if connected to the correct user body.

If the parameter or quantity measured during the sensing action is user-specific, for example, a DNA sample, blood type, etc., the sensor could be configured for generating the lock signal if the device is physically connected to a non-intended user as determined from the measured parameter.

If the sensor is adapted for measuring multiple different parameters, such as lead impedance, intracardiac signals and device temperature, the access signal could be generated if all of these different measured parameter values correspond to their respective default values or if at least a set of the measured parameter values corresponds to the default values. Correspondingly, the lock signal could be generated if any of the measured parameter values diverges from the determined default value or value range or only if at least two or more of the measured values diverge from the default value (ranges).

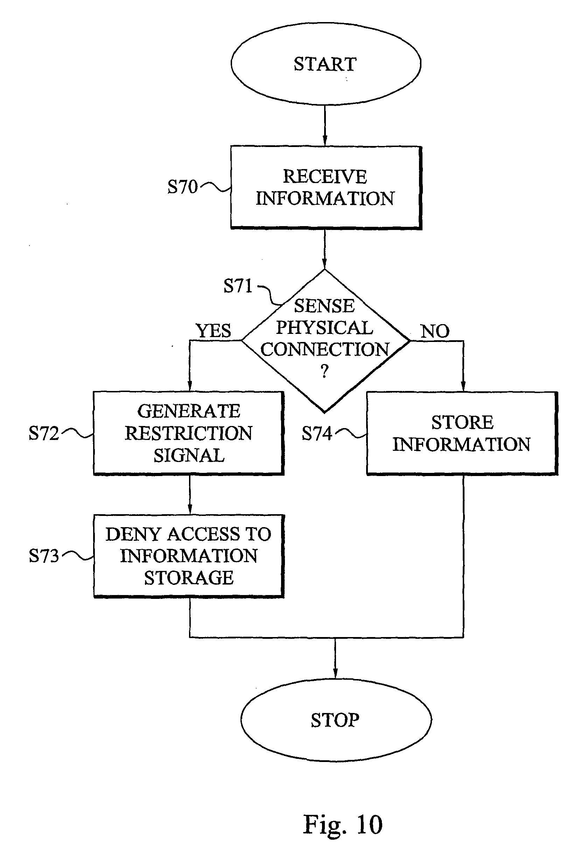

Fig. 10 is a flow diagram of an embodiment of the information management method of the present invention. The method starts in step S70, where the device receives information or data to be stored in the device or used for updating a setting of the device. In this particular embodiment, the information will not be entered if the device is physically connected to the user body, preferably if the device is implanted in the user body. Therefore, a next step S71 senses whether the device is physically (mechanically and optionally functionally) connected to, preferably implanted in, the user body. If the device is physically connected (implanted), a restriction or prohibition signal is generated in step S72. The information manager of the device is then responsive to this restriction signal and prevents permanent entering of the received information in step S73, e.g. by denying access to the information storage.

This embodiment is particularly useful for preventing entering of erroneous information or updating information that should be static, such as device serial and model number. This means that once the device has been connected to the correct user, such static data should not be updatable or changed by adding new corresponding data to the device.

If the device, however, is not connected to the user body, such as following manufacturing but before connection to the user, the sensing step S71 detects the lack of physical device-body connection. In such a case, an optional access signal can be generated and the received information can be stored in the device in step S74 based on the access signal. The method then ends.

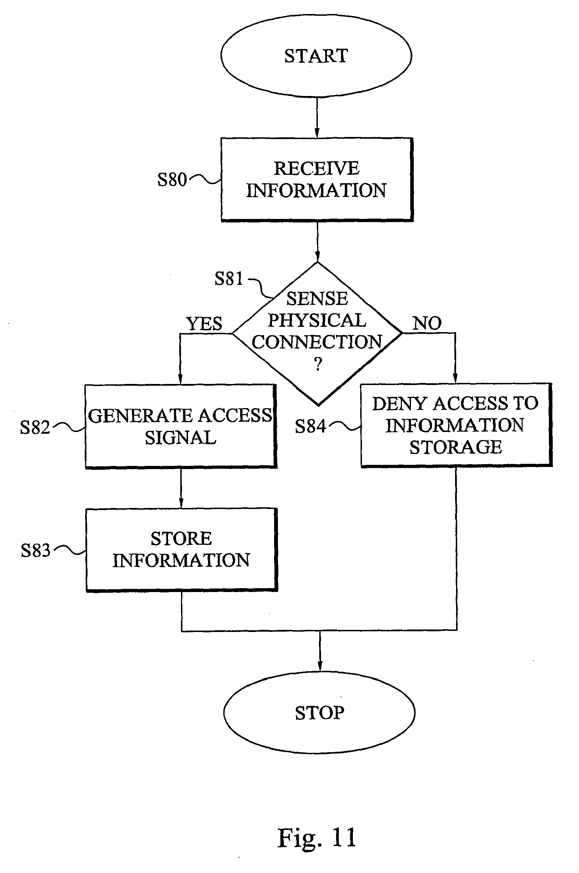

Fig. 11 is a flow diagram of a further example of the information management method not forming part of the present invention. The method starts step S80, where the device receives information or data to be stored in the device or used for updating a setting of the device. In this particular embodiment, the information will only be entered if the device is physically connected to the user body, preferably if the device is implanted in the user body. Therefore, a next step S81 senses whether the device is physically (mechanically and optionally functionally) connected to, preferably implanted in, the user body. If the device is physically connected (implanted), an access signal is generated in step S82. The information manager of the device is then responsive to this access signal and enters the received information in a storage or some other dedicated location of the device in step S83.

In the case no physical connection is sensed in step S81, the sensor could generate a restriction signal. The information manager of the device is then responsive to this restriction signal and prevents permanent entering of the received information in step S83, e.g. by denying access to the information storage. The method then ends.

The two methods described above and disclosed in Figs. 10 and 11 may complement each other. Thus, certain information and data should only be allowed to enter the device when physically connected to the user (Fig. 11), whereas no information storage or update should be allowed for other data types (Fig. 10). In such a case, the device preferably includes functionality for determining, once information is received, whether the received information is allowed to be entered if the device is correctly connected and worn by the user or entered only when not worn by (preferably implemented in) the user. This means that the flow diagrams of Figs. 10 and 11 can be combined so that once information is received in a first step (S70/S80), the device analyzes the received information. If the information is of a type that is stored only in non-worn devices the method continues to step S71 of Fig. 10 otherwise the method continues to step S81 of Fig. 11.

The methods according to the present invention may be implemented as software, hardware, or a combination thereof. A computer program product implementing the methods or a part thereof comprises software or a computer program run on a general purpose or specially adapted computer, processor or microprocessor. The software includes computer program code elements or software code portions that make the computer perform the methods using at least one of the steps previously described in Figs. 3 to 10. The program may be stored in whole or part, on, or in, one or more suitable computer readable media or data storage means such as a magnetic disk, CD-ROM or DVD disk, hard disk, USB memory, magneto-optical memory storage means, in RAM or volatile memory, in ROM or flash memory, as firmware, or on a data server.

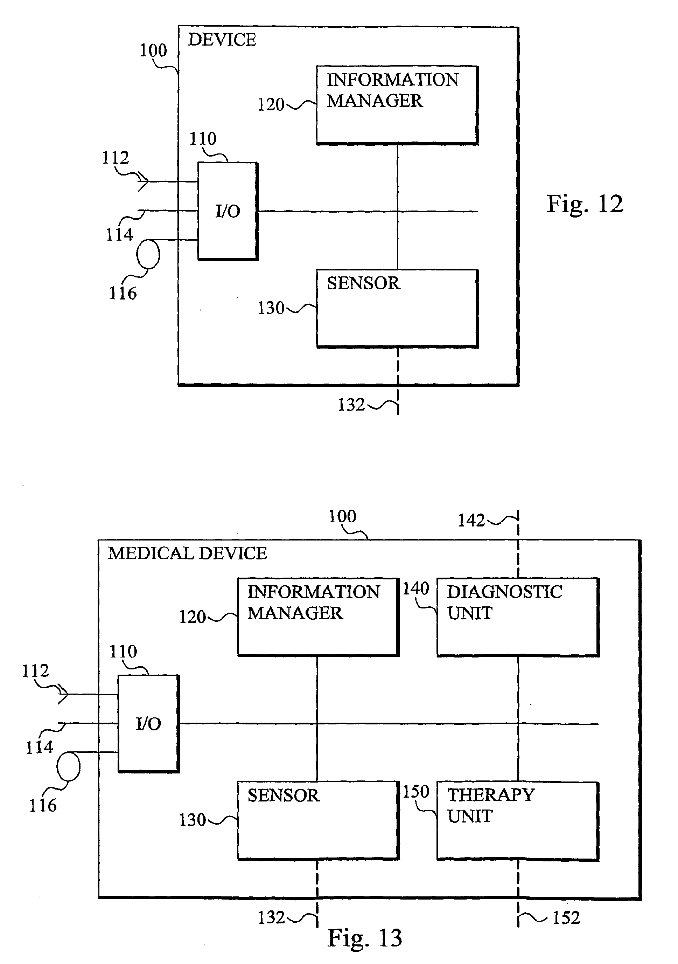

Fig. 12 is a schematic block diagram of a device 100 adapted for physical connection to a body of a user according to an embodiment of the present invention. The device 100 generally includes means and equipment 110 for communicating with at least one external communications unit. In the figure, this equipment has collectively been represented by a general input and output (I/O) unit 110. It is anticipated by the present invention that this I/O unit 110 includes those functionalities required for conducting wired or wireless communication with the at least one external unit and processing incoming and outgoing data, such as transmitter/receiver (transceiver), modulator/demodulator, coder/decoder, etc. The I/O unit 110 can in turn be connected to an antenna arrangement 112 used for transmitting and receiving radio packets to and from the external unit, respectively. However, the I/O unit 110 could also or alternatively use other forms of wireless techniques than radio packets when communicating with the external device. The I/O unit 110 could for example use an inductive antenna 116 for external wireless communication. In a further application, the I/O unit 110 includes a port 114 in which a wire can be inserted for conducting wired communication with the external unit.

The I/O unit 110 is in particular arranged for receiving information requests from external units, where the requested information is stored in the device 100 or is generated internally in the device 100. This requested information is provided by a manager 120 arranged connected to the I/O unit 110 in the device 100. Once the requested information has been provided by the manager 120, the I/O unit 110 transmits it to the requesting external unit.

The I/O unit 110 can also be arranged for receiving information and data to be entered in the device 110. In such a case, the I/O unit 110 preferably forwards the received data to the manager 120 for processing and storage.

In either case, the device 100 comprises a sensor 130 for sensing whether the device 100 is physically connected, such as mechanically connected, to the body of the user. The sensor 130 is further arranged for generating a signal, based on the connection sensing, representative of whether the device 100 is actually physically connected to the user body. For these sensing purposes the sensor 130 may include or be connectable to a probe, lead or similar equipment 132 that is in (physical) connection to the body and is employed for measuring or collecting a physical parameter value from the user body. This parameter value is used by the sensor 130 for determining whether a physical body-device connection is present or not. In this determination process, the sensor 130 preferably compares the measured parameter value with a pre-defined default value or default value range, which is representative of the presence of such a physical connection. If the measured value is equal to the default value, differs from the default value with no more than a maximum hysteresis value or falls within the default value range, the sensor 130 generates a signal that is representative of the presence of the body-device connection. Correspondingly, if the measured value diverges too much from the default value or is outside of the default range, the sensor 130 could generate a signal that is representative of the lack of correct body-device connection.

The sensor 130 of the present invention, thus, includes functionality for determining, based on a measured value, whether the device-body connection is present. This means that the sensor 130 includes data processors for processing the measured and collected data/signal and for determining based on the processed data and preferably default data whether the device 100 is connected to the user body. In an embodiment, the sensor 130 may also include the actual equipment 132 (probe, lead, etc.) used for collecting the parameter data from the user body. In other embodiments this data collecting equipment 132 is connected to the sensor 130 and forwards measurement results to the sensor 130 for processing. The term "sensor" should therefore be interpreted broadly in the present document to include any data processing unit that can process measured data for the purpose of sensing a possible physical device-body connection.

The sensor 130 can be implemented for performing the connection sensing once the I/O unit 110 receives an information request or information to be entered in the device. The I/O unit 110 then preferably informs the sensor 130 of such a reception and urges it to perform a new sensing operation. Alternatively, or in addition, the sensor 130 can be implemented for performing the sensing operation (optionally performing parameter measuring, processing of measurement results and determining the presence or absence of a physical device-body connection) at multiple time instances, preferably based on a pre-defined sensing schedule. The results for multiple such sensing operations can be used in the signal generation, a single access/restriction/lock signal is generated after each sensing occasion or a signal is generated only when required by the information manager 120.

The sensor 130 is connected with the information manger 120. This information manager 120 is provided in the device 100 for managing information and data associated with the user and/or the device 100. The manager 120 is responsive to the signal generated by the sensor 130 and performs the information management based on the generated signal.

The sensor 130 generates a restriction signal if a physical body-device connection was sensed. Correspondingly, an access signal will be generated if no such connection can be sensed. This embodiment is particularly advantage in connection with entering and updating "static" information of the device 100 and is preferably only applicable to such information types, such as fixed serial and model number, fixed patient identifier, etc. The units 110, 120 and 130 of the device 100 may be provided as hardware, software or a combination of hardware and software.

In a preferred implementation, the sensor of the device is arranged for sensing whether the device is functionally connected to the user body. In such a case, the device includes equipment for exerting a defined function to the user body, such as a diagnostic, therapeutic, monitoring or medical function. The sensor then includes or is connected to a probe, measurement equipment, etc. for determining whether the device is actually connected to the user body and exerts its intended function correctly. Fig. 13 is a schematic block diagram of a medical device 100 having such a therapeutic/diagnostic function. For example, the medical device 100 can include a diagnostic unit 140 for measuring or monitoring at least one physical parameter of the user body. This parameter can be a parameter measured in the blood system of the user, such as blood glucose level, a parameter measured in connection with an organ or tissue, such as intracardiac signal, pulmonary/respiratory activity, brain and/or spinal activity, kidney-related parameters, liver-related parameters, etc, or any other parameter that can be of diagnostic or therapeutic value and which is preferably measured and monitored during the daily life of the user.

The diagnostic unit 140 comprises or is connected to a probe or other diagnostic data collecting equipment 142 that is inserted into or connected to the user.

In addition, or alternatively, the medical device 100 comprises a therapy unit 150 arranged for exerting a defined therapeutic action when needed. For example, the therapy unit 150 could have pacemaker or defibrillator activity by delivering, when needed, a voltage to the patient's heart by means of a lead or probe 152 inserted into the heart. Other examples of therapy unit 150 according to the present invention include different drug pumps and neurological stimulators.

The medical device 100 can be equipped with either of these units 140, 150 but may also contain both units 140, 150, such as for a combined insulin pump and blood glucose monitoring device or a pacemaker. The sensor 130 is then preferably arranged for sensing whether the medical device 100 is functionally connected to the user body in such a way that the diagnosing unit 140 can perform its diagnosing function in the body and/or the therapy unit 150 can perform its therapy function to the body.

The sensor 130 can receive measurement results and other data from the diagnostic 140 and/or therapy 150 unit for processing this data for the purpose of determining whether the device 100 is correctly functionally connected to the user body. In such a case, the sensor 130 does not itself need or must connected to the probe 132 but could instead utilize the probes/leads 142, 152 of the diagnostic 140 and therapy 150 unit for the data gathering task.

The operation of the I/O unit 110 and the information manager 120 is similar to the corresponding units described above in connection to Fig. 12 and will not be described further herein.

The units 110, 120, 130, 140 and 150 of the device 100 may be provided as hardware, software or a combination of hardware and software.

The device is an implantable device and more preferably an implantable medical device 100 as illustrated in Fig. 14. The device 100 is thus adapted for surgically connection to the user body and the device 100 can preferably not be removed from the user body with less than a surgical operation.

The device 100 is preferably implanted in the user body for performing a diagnostic and/or therapeutic function. Typical examples of such implanted medical devices include the previously mentioned pacemakers, implantable cardioverters, implantable defibrillators and neurological stimulators.

The implanted device 100 includes an I/O unit 110 and information manager 120, the operation of which has previously been described in detail. The I/O unit 110 is, however, typically only limited to wireless data transmission in form of RF transmission using an RF antenna 112 and/or inductive data transmission by means of an inductive antenna 116.

In Fig. 14, the diagnostic 140 and therapy 150 units have non-limitedly been illustrated as constituting part of the sensor 130 of the present invention. The reason for this is that in this embodiment, the sensor 130 is preferably arranged for sensing whether the implantable device 100 is actually implanted in the user body. The sensor 130 preferably utilizes information collected by the diagnostic unit 140 and/or therapy unit 150 (it is anticipated by the present invention that the sensor 130 and the implantable device 100 could be equipped with either of these units 140, 150) for determining whether the device 100 is still implanted.

A typical example of such sensed parameters that are particularly adapted for exerting a heart monitoring and/or therapy function include: i) device temperature (should be equal to body temperature, about 37 °C, when implanted), ii) lead impedance of the lead 132, 134 inserted into the patient's heart (should have normal lead impedances), iii) oxygen levels in the heart, iv) intracardiac electric signal, v) signals detected from the operation of the valves, vi) measured heart sound, vii) electrocardiogram (should have correct PQRST pattern), etc.

Lead impedance is a particular suitable parameter to use when determining whether the IMD is actually implanted in a patient body. Prior to implantation, the impedance of the electrode system comprising the leads 132, 134 is very high, typically so high that the impedance does not impose any load on the IMD 100. After the leads 132, 134 are connected to the heart of the patient, the impedance normally drops to about 500 ohms. Thus, setting a threshold value for determining whether the IMD 100 is implanted or not could be one or a few kohms.

In an IMD embodiment having a therapy unit 150 generating stimulation pulses to e.g. a heart of a patient, the impedance measurement can be performed by measuring the time required to recharge a reservoir capacitor of the therapy unit 150 to a pre-designated stimulation voltage between each stimulation pulse. When not implanted, the lead impedance is very high so discharge of the storage capacitor between two stimulation pulses is determined only by leakage currents in the capacitor itself and possibly in other components of the therapy unit 150. Recharging of the reservoir capacitor to the pre-designated stimulation voltage is therefore very fast. After implantation, however, the impedance is much lower so a far bigger charge is discharged through the electrode system of the therapy unit 150 for each stimulation pulse. The time required to recharge the storage capacitor between two stimulation pulses therefore becomes much longer. Recharge is normally less than 1 ms before implantation and in the 3-10 ms range after implantation.

In another embodiment, the storage capacitor of the therapy unit 150 can be recharged in steps. Thus, charging takes place for a given period of time and the storage capacitor's voltage is thereafter compared in a comparator to the pre-designated stimulation voltage. If the capacitor voltage is less than the stimulation voltage, the capacitor is connected to a battery of the IMD 100 for additional charging for a second period of time. The capacitor voltage is then again compared to the pre-designated stimulation voltage. This procedure is repeated until the capacitor voltage reaches or exceeds the pre-designated stimulation voltage, whereupon the charging terminates. The periods of time in which the storage capacitor is connected to the battery during the recharging process are preferably of equal length. The number of such recharging periods required to achieve the reference voltage is a measure of the lead impedance and can therefore be used to discriminate whether the IMD 100 is implanted or not.

In still another embodiment, the voltage across the storage capacitor is measured before and after emission of a stimulation pulse by the therapy unit 150. Prior to IMD implantation, when the impedance of the leads 132, 134 is high, the difference in voltage before and after a stimulation pulse is relatively small, whereas this voltage difference is much bigger after the IMD 100 has been implanted and the lead system 132, 134 connected to the patient's heart.

In a preferred embodiment of the invention, the diagnostic 140 and/or therapy 150 units are connected via a lead or wire 132 to a probe 134 used for collecting physiological data and measuring physiological parameters in the body of the patient in which the IMD 100 is implemented. This physiological data can e.g. be IEGM (intracardiac electrogram), ECG (electrocardiogram), electromyography (EMG), electroencephalography (EEG), electrooculography (EOG), event marker, respiration, blood pressure and/or oximetry-related data. This collected physiological data can, in addition from being used by the sensor 130 for determining whether the device 100 is implantable, be (temporarily) stored by the information manager 120 and constitutes sensitive medical data (PHI).

This collected medical data can then be transmitted, using the I/O unit 110, to an external unit for storing, processing and/or display. This data transmission is, however, according to a preferred embodiment conditioned to only take place if the implanted device 100 is still implanted in the body of the user.

The units 110, 120, 130, 140 and 150 of the device 100 may be provided as hardware, software or a combination of hardware and software.

It is anticipated that the teachings of the present invention can be applied other types of implanted devices that does generally not have a diagnostic or therapeutic function. For example, implanted memory chips having the functionality of storing (sensitive) data associated with the host and can also be regarded as an implanted device according to the present invention. In such a case, the diagnostic and therapeutic units illustrated in Fig. 14 are omitted so that the implanted memory chip comprises the I/O unit, information manager and sensor. The retrieval of data from the memory chip is then preferably only allowed if the chip is implanted in the host, as determined by the sensor.

Another example is an implantable ID card that comprises an identifier of the user in which the ID card is implanted. Normally, this user identifier stored in the card should not be changeable after implantation. In such a case, the sensor can generate a restriction signal as long as the ID card is implanted in the user body and possible a lock signal if the sensor, after it has detected a correct card implantation, detects that the ID card no longer is implanted. Alternatively, the sensor generates an access signal if the ID card is correctly implanted in the body of the user and an entering of the user identifier is to take place. The information manager then adds this new user identifier in a storage location of the card based on the access signal. Once the manager has added the user ID in the implanted ID card it can become, at least temporarily, blocked or insensitive to new information addition requests. This means that the user ID can only be entered in the ID card if implanted in the user and the entered user ID cannot be changed, at least as long as the card is still implanted in the user body. This implanted ID card can of course also use the restrictive information retrieval function described above for the implanted memory chip, i.e. the stored user ID is only accessible for external unit if the ID card is implanted.

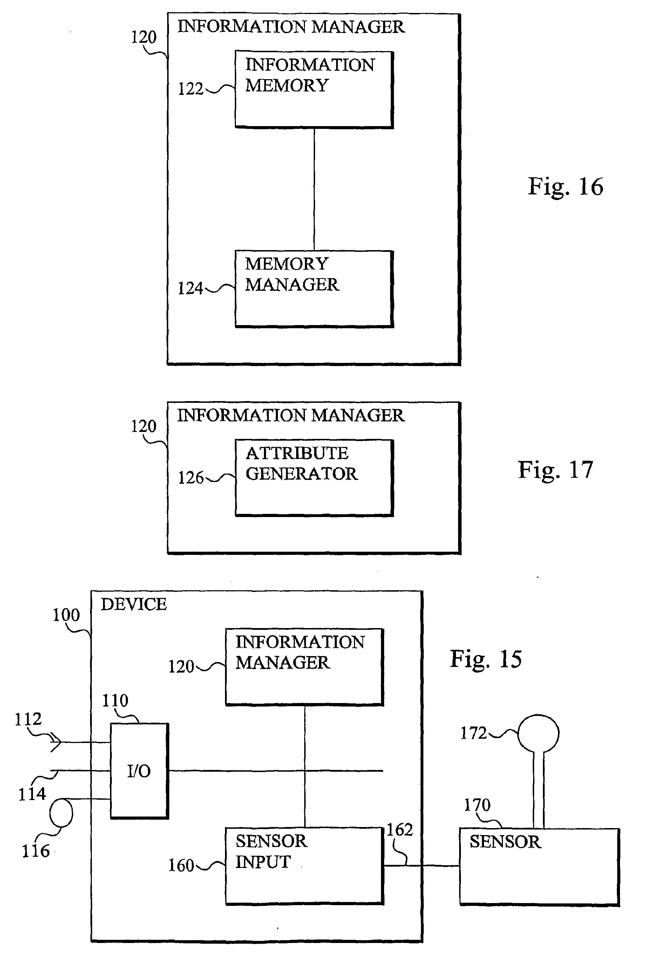

Fig. 15 is a schematic block diagram of another embodiment of a device 100 adapted for physical connection to a user body according to the present invention. This device embodiment comprises, as the previously described embodiments, an I/O unit 110 and an information manager 120, the operation of which corresponds to the discussion presented above. The device 100, however, does not include the sensor 170 used for sensing whether the device 100 is physically connected to the user body. Instead this sensor 170 is provided outside of the device 100 but is connectable 162 thereto through a sensor input 160. This solution can be useful when it is desired to measure and sense a parameter in the user body at a location which is not in the closest vicinity of the device 100. The sensor 170, which preferably includes a sensor probe or lead 172, can then be arranged at this desired sensing location and is then in wired connection 162 with the sensor input 160. For example, the device 100 could be an externally arranged drug pump, whereas the sensor 170 is implanted in the user body for measuring the body temperature. In another example the device 100 is an implantable medical device and the sensor 170 is likewise implanted but at in a different position in the user body.

The actual operation of the external sensor 170 and sensor 172 basically corresponds to the sensor operation of the previously described embodiments. The sensor 170 generates a sensing signal which is transmitted using the wire 162 to the sensor input 160 and where this sensing signal is representative of whether there is a physical connection between the device 100 and the user body. The sensor input 160 forwards this signal to the information manager 120, which performs its information managing function based on the signal.

The input sense signal could be the previously described access, restriction or lock signal. In such a case, the sensor input 160 typically only forwards the signal to the manager 120 without any extensive signal processing. However, in another embodiment, the external sensor 170 merely measures and collects parameter data and forwards it to the sensor input 160. In such a case, the input 160 preferably comprises or is at least connected to functionality for processing the received parameter data for the purpose of determining whether there is a physical device-body connection present or not based, at least partly, on the parameter data. The input 160 (or separate processing unit) then generates an access/restriction/lock signal based on the processed parameter data and forwards the generated signal to the information manager 120.

The units 110, 120 and 160 of the device 100 may be provided as hardware, software or a combination of hardware and software.

Fig. 16 is a schematic block diagram of an information manager embodiment that can be used in any of the devices illustrated by Figs. 12 to 15. This information manager 120 comprises or has access to an information memory 122, which constitutes a storage location for (at least temporarily) storing information and data associated with at least one of the device and the user to which the device is connected. A memory manager 124 of the information manager 120 is connected to this memory 122 and manages data stored therein based on the signal provided from the sensor or sensor input of the device. In a preferred implementation, the memory manager 124 receives a request for information stored in the memory 122 and retrieves this requested information if it has received an access signal from the sensor/sensor input, i.e. if the device is physically connected to the user body. The retrieved information is then forwarded by the manager 124 to the I/O unit for transmission to a requesting external unit.

In a further preferred implementation, the memory manager 124 is also responsive to a lock signal, which represents the lack of physical device-body connection. In such a case, the memory manager 124, once it receives a lock signal, will no longer retrieve any information from the memory 122 even if it subsequently receives an information request. This lock of the memory manager 124 could be permanent or it could be reversible, i.e. unlocked by resetting the memory manager 124. In the latter case, the resetting is preferably only performable by a trusted party, such as the user's physician or the manufacturer of the device.

It is anticipated by the present invention that the memory manager 124 can also be responsive to the previously described restriction signal and, in addition, be used for entering, based on the signal from the sensor/sensor input, information in the memory 122.

The information manager 120 can constitute a tamper-resistant device or at least the information memory 122 can be a tamper-resistant memory for making it harder for a malicious person to access information stored therein.

The unit 124 of the information manager 120 may be provided as hardware, software or a combination of hardware and software. The units 122 and 124 may all be implemented in the manager 120. Alternatively, a distributed solution with at least one of the unit 122 and 124 implemented elsewhere in the device can be used.

Fig. 17 is a schematic block diagram of another information manager example. This information manger 120 comprises an attribute generator 126, which is arranged for, upon request, generating security attributes, such as security credentials, temporary session keys, temporary authentication tokens, etc., which can be used for accessing patient records, node-node communication and other operations that requires authentication of the participating parties and/or other security operations.

This attribute generator 126 is responsive to the access/restriction/lock signal provided from the sensor or sensor input of the device. In a preferred implementation, the generator 126 can generate a security attribute first once it has received an access signal. Thus, sensitive security attributes will then only be available if the device is correctly implemented on the body of the user. Correspondingly to the memory manager of Fig. 16, the attribute generator 126 may also be responsive to a lock signal, which causes a, at least temporary, lock of the generator 126. This will prevent any fraudulent retrieval of security attribute is the user is not present and prevent obtaining the attributes if the device has been removed (explanted) from the intended user.

The unit 126 of the information manager 120 may be provided as hardware, software or a combination of hardware and software.

It will be understood by a person skilled in the art that various modifications and changes may be made to the present invention without departure from the scope thereof, which is defined by the appended claims.

## Claims

1. A device (100) adapted for physical connection to a body (10) of a user, said device (100) comprises:
a receiver (110) for receiving, from an external communication unit (200), information representing serial and model number of said implantable medical device (100) or user identifier of said user; and
a memory (122) for storing said information;
**characterized in that** said device (100) is an implantable medical device (100) adapted for implantation into said body (10), said implantable medical device (100) further comprises:
a sensor (130) for sensing whether said implantable medical device (100) is implanted in said body (10) based on a comparison of a measured parameter value and a pre-defined default value range representative of said implantable medical device being implanted in said body (10) and for generating a restriction signal if said implantable medical device (100) is implanted in said body (10); and
an information manager (120) connected to said sensor (130) and arranged for selectively entering said information received by said receiver (110) in said memory (122) and responsive to said restriction signal by preventing entering of said information received by said receiver (110) in said memory (122) based on said restriction signal.

2. The device according to claim 1, **characterized in that** said sensor (130) is arranged for sensing a parameter value selected from a group consisting of temperature of said implantable medical device, lead impedance of an electrode system, oxygen levels in a heart (15) of said user, intracardiac electric signals, measured heart sound, electrocardiogram.

3. The device according to claim 1 or 2, **characterized in that**
said sensor (130) is arranged for generating an access signal if said implantable medical device (100) is not implanted in said body (10); and
said information manager (120) is responsive to said access signal by storing said information received by said receiver (110) in said memory (122) based on said access signal.

## Patentansprüche

1. Vorrichtung (100), die für eine physische Verbindung mit einem Körper (10) eines Benutzers ausgelegt ist, wobei die Vorrichtung (100) Folgendes umfasst:
einen Empfänger (110) zum Empfangen, von einer externen Datenübertragungseinheit (200), von Informationen, die die Serien- und Modellnummer des implantierbaren Medizinprodukts (100) oder eine Benutzerkennung des Benutzers darstellen; und
einen Speicher (122) zum Speichern der Informationen;
**dadurch gekennzeichnet, dass** es sich bei der Vorrichtung (100) um ein implantierbares Medizinprodukt (100) handelt, das für die Implantation in den Körper (10) ausgelegt ist, wobei das implantierbare Medizinprodukt (100) ferner Folgendes umfasst:
einen Sensor (130) zum Erfassen, ob das implantierbare Medizinprodukt (100) in dem Körper (10) implantiert ist, auf der Grundlage eines Vergleichs von einem gemessenen Parameterwert mit einem vorher festgelegten Standardwertebereich, der bedeutet, dass das implantierbare Medizinprodukt in dem Körper (10) implantiert ist, und zum Erzeugen eines Beschränkungssignals, wenn das implantierbare Medizinprodukt (100) in dem Körper (10) implantiert ist; und
einen Informationsmanager (120), der mit dem Sensor (130) verbunden und so angeordnet ist, dass er gezielt die von dem Empfänger (110) empfangenen Informationen in den Speicher (122) eingibt, und der auf das Beschränkungssignal reagiert, indem er das Eingeben der von dem Empfänger (110) empfangenen Informationen in den Speicher (122) auf der Grundlage des Beschränkungssignals verhindert.

2. Vorrichtung nach Anspruch 1, **dadurch gekennzeichnet, dass** der Sensor (130) so angeordnet ist, dass er einen Parameterwert erfasst, der aus einer Gruppe ausgewählt ist, die aus der Temperatur des implantierbaren Medizinprodukts, der Leitungsimpedanz eines Elektrodensystems, dem Sauerstoffgehalt in einem Herzen (15) des Benutzers, intrakardialen elektrischen Signalen, gemessenen Herztönen und einem Elektrokardiogramm besteht.

3. Vorrichtung nach Anspruch 1 oder 2, **dadurch gekennzeichnet, dass**
der Sensor (130) so angeordnet ist, dass er ein Zugangssignal erzeugt, wenn das implantierbare Medizinprodukt (100) nicht in dem Körper (10) implantiert ist; und
der Informationsmanager (120) auf das Zugangssignal reagiert, indem er die von dem Empfänger (110) empfangenen Informationen auf der Grundlage des Zugangssignals in dem Speicher (122) speichert.

## Revendications

1. Dispositif (100) adapté pour le raccordement physique à un corps (10) d'un utilisateur, ledit dispositif (100) comprenant :
un récepteur (110) pour recevoir, d'une unité de transmission extérieure (200), des informations représentant un numéro de série et de modèle dudit dispositif médical implantable (100) ou un identifiant d'utilisateur dudit utilisateur ; et
une mémoire (122) pour enregistrer lesdites informations ;
**caractérisé en ce que** ledit dispositif (100) est un dispositif médical implantable (100) adapté pour être implanté dans ledit corps (10), ledit dispositif médical implantable (100) comprenant en outre :
un capteur (130) pour détecter si ledit dispositif médical implantable (100) est implanté dans ledit corps (10) sur la base d'une comparaison d'une valeur de paramètre mesurée avec une plage de valeurs par défaut prédéfinie représentative du fait que le dispositif médical implantable est implanté dans ledit corps (10) et pour générer un signal de restriction si ledit dispositif médical implantable (100) est implanté dans ledit corps (10) ; et
un gestionnaire d'informations (120) raccordé audit capteur (130) et agencé pour saisir de manière sélective dans ladite mémoire (122) lesdites informations reçues par ledit récepteur (110) et réagissant audit signal de restriction en empêchant la saisie dans ladite mémoire (122) desdites informations reçues par ledit récepteur (110), sur la base dudit signal de restriction.

2. Dispositif selon la revendication 1, **caractérisé en ce que** ledit capteur (130) est agencé pour détecter une valeur de paramètre sélectionnée dans un groupe constitué de la température dudit dispositif médical implantable, l'impédance de dérivation d'un système d'électrodes, le niveau d'oxygène dans un coeur (15) dudit utilisateur, des signaux électriques intracardiaques, le bruit du coeur mesuré, un électrocardiogramme.

3. Dispositif selon la revendication 1 ou 2, **caractérisé en ce que**
ledit capteur (130) est agencé pour générer un signal d'accès si ledit dispositif médical implantable (100) n'est pas implanté dans ledit corps (10) ; et
ledit gestionnaire d'informations (120) réagit audit signal d'accès en enregistrant dans ladite mémoire (122) lesdites informations reçues par ledit récepteur (110), sur la base dudit signal d'accès.
